# EUROPEAN PATENT APPLICATION

(11) **EP 4 105 320 A1**
(43) Date of publication of application: **21.12.2022**
(21) Application number: 20918987.7
(22) Date of filing: 26.02.2020
(51) Int. Cl.: C12N 5/09

(54) **CULTURE MEDIUM FOR PRIMARY CELLS OF ESOPHAGEAL SQUAMOUS CARCINOMA, AND CULTIVATION METHOD THEREFOR**

(30) Priority: 11.02.2020 CN 202010086392
(71) Applicant: Precedo Pharmaceuticals Co., Ltd., Hefei, Anhui 230094 (CN)
(72) Inventor: LIU, Qingsong, Hefe, Anhui 230031 (CN); HU, Jie, Hefei, Anhui 230031 (CN); CHEN, Cheng, Hefei, Anhui 230031 (CN); WANG, Wenchao, Hefei, Anhui 230031 (CN); WANG, Li, Hefei, Anhui 230031 (CN)
(74) Representative: Abel & Imray LLP
(86) International application number: PCT/CN2020/076736
(87) International publication number: WO 2021/159560

(57) **Abstract**

Provided are a culture medium and cultivation method of rapidly expanding primary cells of esophageal squamous carcinoma in vitro, and the use thereof in screening drugs. The culture medium comprises an initial culture medium selected from DMEM/F12, DMEM, F12, or RPMI-1640, a Rho protease inhibitor, an antibiotic, insulin, an N2 additive, insulin-like growth factor 1, a non-essential amino acid, and optionally, hydrocortisone, optionally, glutamine, and optionally, bovine pituitary extract.

## Description

### Technical Field

The invention relates to the technical field of biology, in particular to a culture medium and cultivation method of rapidly expanding primary cells of esophageal squamous carcinoma in vitro.

### Background of the Invention

Currently, tumor is the main cause of death in China, both in cities and in rural areas. Esophageal cancer is one of the most common gastrointestinal malignancies in the world. According to the latest statistics from the National Cancer Center, esophageal cancer ranks fourth among the top ten malignant tumors in men and ranks third in women. In many regions of the world, the local incidence rate has increased, while China is a high incidence region of esophageal cancer, with an average annual death toll of about 150,000, accounting for 21.8% of the national cancer mortality, ranking the fourth among the cancers that cause the most deaths (non-patent document 1). In the pathological classification of esophageal cancer, there are obvious differences between foreign countries and China: more than 90% of esophageal cancers in foreign countries are adenocarcinomas, while more than 90% in China are squamous carcinomas. As NCCN guidelines are established based on foreign case studies, there are certain differences in the use of NCCN to guide the medication of domestic esophageal cancer patients. Therefore, it is necessary to establish the primary cell sample bank of Chinese population, to be used to study the pathogenesis of esophageal cancer in vitro and develop new drugs for the treatment of esophageal cancer. In this process, it is particularly important to cultivate the primary cells of esophageal cancer.

At present, in the cultivation of the primary cells of esophageal cancer, there is a known method of isolating tumor cells and then directly culturing the same in a medium containing fetal bovine serum (non-patent document 2); in recent years, a method of using conditional reprogramming process to culture epithelial-derived cells has also been reported, in which it is mentioned that the method can be used to culture and expand primary cells of esophageal cancer (non-patent document 3); subsequently, it is reported in the literature that the intraoperative samples of esophageal cancer were digested by trypsin and cultured by conditional reprogramming method (non-patent document 4). Recently, literatures have reported the cultivation method of using the commercial culture medium KSFM with additional factors (non-patent document 5), and the cultivation method of using organoid technology (non-patent document 6).

However, the above method of non-patent document 2 cannot be used for stably culture for a long time; the cultivation periods of non-patent document 3 and non-patent document 4 are relatively long; the method of non-patent document 5 requires higher costs; the reagents used for organoid culture in non-patent document 6 are too expensive, and the operation in the cultivation process is complex, which is unfavourable for extensive application.

In addition, as the pathological type of esophageal cancer in foreign countries is mainly esophageal adenocarcinoma, and the methods reporded in foreign literatures are basically based on esophageal adenocarcinoma, they are not suitable for culturing esophageal squamous carcinoma. Therefore, it is necessary to develop a culture medium and cultivation method suitable for culturing primary cells of esophageal squamous carcinoma.

### Prior art documents

### Non-patent documents

Non-patent documents 1: Freddie Bray, BSc, MSc, PhD; Jacques Ferlay, ME, et al. Global Cancer Statistics 2018. CA CANCER J CLIN 2018; 68:394-424;
Non-patent documents 2: Karin J. Purdie, Celine Pourreyron, and Andrew P. South. Isolation and Culture of Squamous Cell Carcinoma lines. Cancer Cell Culture: Methods and Protocols, Second Edition, Methods in Molecular Biology, vol. 731, 2011, 151-159;
Non-patent documents 3: Xuefeng Liu, Ewa Krawczyk, et al. Conditional reprogramming and long-term expansion of normal and tumor cells from human biospecimens. Nature Protocols, VOI.12 NO.2, 2017, 439-451;
Non-patent documents 4: Todd J. Jensen, Christopher Foster, et al. Conditional Reprogramming of Pediatric Human Esophageal Epithelial Cells for Use in Tissue Engineering and Disease Investigation. Journal of Visualized Experiments, March 2017, 121, e55243;
Non-patent documents 5: Yuta Kasagi,et al. The Esophageal Organoid System Reveals Functional Interplay Between Notch and Cytokines in Reactive Epithelial Changes.Cellular and Molecular Gastroenterology and Hepatology Vol. 5, No. 3, 2018, 333-352;
Non-patent documents 6: Xiaodun Li, Hayley E. Francies, et al. Organoid cultures recapitulate esophageal adenocarcinoma heterogeneity providing a model for clonality studies and precision therapeutics. NATURE COMMUNICATIONS (2018) 9:2983-2995.

### Summary of the Invention

In order to solve the above problems in the prior art, the invention provides a culture medium and a cultivation method for rapidly expanding primary cells of esophageal squamous carcinoma in vitro.

One aspect of the invention is to provide a culture medium for primary cells of esophageal squamous carcinoma, which comprises an initial culture medium, the following components (1) - (6), and optionally the following components (7) - (9), wherein the initial culture medium can be, for example, DMEM/F12, DMEM, F12 or RPMI-1640, preferably DMEM/F12.
(1) Rho protease inhibitor, which is selected from one or more of Y27632, Hydroxyfasudil and GSK429286A; in case of Y27632, having a concentration within the range of 2.5-40 µM, preferably 5-20 µM, more preferably 10 µM; in case of Hydroxyfasudil, having a concentration within the range of 2-32 µM, preferably 4-16 µM, more preferably 8 µM; and in case of GSK429286A, having a concentration within the range of 2-32 µM, preferably 4-16 µM, more preferably 8µM;
(2) antibiotics, which is selected from one or more of streptomycin / penicillin, Amphotericin B and Primocin; in case of streptomycin / penicillin, with streptomycin having a concentration within the range of 25-400 µg/mL, preferably 50-200 µg/mL, more preferably 200 µg/mL,with penicillin having a concentration within the range of 25-400 U/mL, preferably 50-200 U/mL, more preferably 200 U/mL; in case of Amphotericin B, having a concentration within the range of 0.25-4 µg/mL,preferably 0.5-2 µg/mL, more preferably 1 µg/mL; and in case of Primocin, having a concentration within the range of 25-400 mg/mL, preferably 50-200 mg/mL, more preferably 100 mg/mL; the antibiotic is preferably Primocin;
(3) insulin, having a concentration within the range of 2.5-40 µg/mL,preferably 10-40 µg/mL, more preferably 20 µg/mL;
(4) N2 additive, having a volume ratio to the culture medium of 1:400-1:25, preferably 1:100-1:25, more preferably 1:50;
(5) insulin-like growth factor 1 (IGF-1), having a concentration within the range of 2.5-40 ng/mL, preferably 2.5-10 ng/mL, more preferably 5 ng/mL;
(6) non-essential amino acid, which is selected from one or more of glycine, alanine, asparagine, aspartic acid, glutamic acid, proline and serine, with a total concentration within the range of 50-400 µM, preferably 100-400 µM, more preferably 400 µM;
(7) hydrocortisone, having a concentration within the range of 0-1.6 µg/mL, preferably 0.2-0.8 µg/mL,more preferably 0.4 µg/mL;
(8) glutamine, having a concentration within the range of 0-8 mM, preferably 1-4 mM, more preferably 2 mM;
(9) bovine pituitary extract, having a concentration within the range of 0-56 µg/mL, preferably 3.5-14 µg/mL,more preferably 7 µg/mL.

Another aspect of the invention is to provide a cultivation method of primary cells of esophageal squamous carcinoma, in which the culture medium for primary cells of esophageal squamous carcinoma is used for culture.

The above-mentioned cultivation method comprises the following steps.

### 1. Isolation of primary cells of esophageal squamous carcinoma

1.1 After being rinsed with tissue cleaning solution, tissue samples, such as endoscopic samples, are added with tissue digestive solution and placed in a constant-temperature shaker (Zhichu Instrument ZQLY-180N) for digestion. For example, 8-14 ml, preferably 12 ml of tissue digestive enzymes are used for digestion; the digestion temperature ranges from 4°C to 37°C, preferably 37°C; the rotation speed for digestion ranges from 200 rpm to 350 rpm, preferably 300 rpm.
1.2 After digestion, the resultant is taken out for observation. If no obvious tissue block is found, the digestion can be terminated; otherwise, it is continued for digestion until the digestion is sufficient. The digestion time ranges from 4 to 8 hours, preferably 6 hours.
1.3 After digestion, the resultant is taken out for centrifugation and the supernatant is discarded, and then is added with initial culture medium containing serum for resuspension to terminate the digestion. The rotation speed for centrifugation ranges from 1200g to 1600g, preferably 1500 rpm; the centrifugation time ranges from 2 to 5 minutes, preferably 3 minutes; the culture medium containing serum can be, for example, DMEM/F12 containing 5% fetal bovine serum.

### 2. Cultivation using the curture medium for primary cells of esophageal squamous carcinoma of the invention

The primary cells of esophageal squamous carcinoma obtained in the above step 1 are resuspended with the curture medium for primary cells of esophageal squamous carcinoma of the invention and counted, which are seeded into a culture dish at a cell density of 5-10 × 10⁴/cm²; at the same time, trophoblastic cells are added into the culture dish at a cell density of 2-3 × 10⁴/cm²; after culturing for 5-7 days, trophoblastic cells are added supplementally at a cell density of 0.5-1 × 10⁴/cm²; the cells are digested for passaging after the cells grow to cover 85% of the culture dish.

Specifically, the formulation of the tissue cleaning solution described in step 1 is: DMEM/F12 basic culture medium containing 100-200 mg/mL Primocin and 2% penicillin / streptomycin solution double antibody. The preparation method of the tissue digestive solution described in step 1 comprises: dissolving 1-2 mg/mL collagenase II, 1-2 mg/mL collagenase IV, 50-100 U/mL deoxyribonucleic acid I, 0.5-1 mg/mL hyaluronidase, 1-3 mM calcium chloride, 1-2% bovine serum albumin in HBSS and RPMI-1640 with a volume ratio of 1:1. The trophoblastic cells described in step 2 is, for example, irradiated NIH-3T3 cells, and the irradiation source is X-ray or γ-ray, preferably γ-ray, with radiation dose of 30-50 Gy, preferably 35 Gy.

### Effects of Invention

In accordance with the invention, by using the improved culture medium for primary cells of esophageal squamous carcinoma, the tissue samples of esophageal squamous carcinoma can be rapidly expanded in a short time, and sufficient numbers of cells can be amplified and obtained in an effective time, which can be used for in vitro high-throughput drug sensitivity tests and for the guidance of precise clinical medication.

Compared with the organoid culture medium, the culture medium for primary cells of esophageal squamous carcinoma of the invention does not need to add expensive factors such as wnt3a, RSPO1 and Noggin, which significantly reduces the costs of culture. In addition, in the organoid culture, the digestion process of organoid is complex; in contrast, the cultivation method of the invention involves simple processing process, and is capable of quickly obtaining a large number of monolayer cells, which can meet the requirements of experiments. Compared with the culture medium used in the traditional conditional reprogramming technology, the period for amplifying to obtain the same quantity of cells in the invention is shorter, and the invention also has a significant proliferation promoting effect for a small number of samples. The culture medium and cultivation method for primary cells of esophageal squamous carcinoma of the invention can significantly improve the success rate of in vitro sustainable expansion of tissue samples of esophageal squamous carcinoma, with an average of more than 85%.

### Description of Drawings

Figure 1 shows the effect of increasing factors in the culture medium for primary cells of esophageal squamous carcinoma on the proliferation of primary cells of esophageal squamous carcinoma.
Figure 2 shows the effect of combination of multiple factors in the culture medium for primary cells of esophageal squamous carcinoma on the proliferation of primary cells of esophageal squamous carcinoma.
Figure 3 shows the effect of adding other factors to the culture medium for primary cells of esophageal squamous carcinoma of the invention on the proliferation of primary cells of esophageal squamous carcinoma.
Figure 4A and Figure 4B are photos (bright field) obtained by observing primary cells of esophageal squamous carcinoma cultured in vitro with a microscope.
Figure 5A and Figure 5B are the identification results of esophageal squamous carcinoma cells cultured in vitro by Wright-Giemsa staining.
Figure 6A and Figure 6B are the identification results of esophageal squamous carcinoma cells cultured in vitro by immunofluorescence staining.
Figure 7 shows the results of the first expansion period and the cell number statistics of the primary cells of esophageal squamous carcinoma using the culture medium for primary cells of esophageal squamous carcinoma of the invention.
Figure 8 is the in vitro expansion curve of the primary cells of esophageal squamous carcinoma using the culture medium for primary cells of esophageal squamous carcinoma of the invention.
Figures 9A to 9P show the effects of different factors in different concentrations in the culture medium for primary cells of esophageal squamous carcinoma on the proliferation of primary cells of esophageal squamous carcinoma.
Figure 10 shows the comparison results of the effects of the present culture medium with the mediums reported in the literature and the commercial medium KSFM on the proliferation of cells.
Figures 11A to 11D show the results of drug screening using using different generations of esophageal squamous carcinoma cells cultured by the culture medium of the invention.

### Detailed Description of the Invention

The invention is further described below through the description of specific embodiments in combination with the drawings. These embodiments are only used to illustrate the invention, and the scope of the invention is not limited to these embodiments.

### Example 1. Cell proliferation promoting effect of the culture medium for primary cells of esophageal squamous carcinoma containing different components

### (1) Preparation of culture medium containing different components

According to the components in Table 1, culture mediums containing different components are prepared for investigating the proliferation promoting effect on esophageal squamous carcinoma cells.

To DMEM/F12 medium (Corning), 10µM Y27632 (MCE) and 100 mg/mL Primocin were added as basic culture medium (hereinafter sometimes referred to as BM), and different factors were added to this basic culture medium. No. 1 medium is obtained by adding insulin (Gibco) to BM, No. 2 medium is obtained by adding hydrocortisone (Sigma) to No. 1 medium, No. 3 medium is obtained by adding non-essential amino acids (NEAA, Gibco) to No. 2 medium, No. 4 medium is obtained by adding glutamine (Gibco) to No. 3 medium, No. 5 medium is obtained by adding bovine pituitary extract (BPE, M&C Gene Technology) to No. 4 medium, No. 6 medium is obtained by adding insulin-like growth factor 1 (IGF-1, Sino Biological) to No. 5 medium, No. 7 medium (hereinafter sometimes referred to as EM) is obtained by adding N2 additive (Gibco) to No. 6 medium, and No. 8 medium is obtained by adding fetal bovine serum (FBS, Gibco) to No. 7 medium.

**Table 1 Preparation of culture medium containing different components (final concentrations are shown)**

| Culture Medium | Component |
|---|---|
| BM | DMEM/F12 + 10 µM Y27632 + 100 mg/mL Primocin |
| No. 1 | BM + 20 µg/mL insulin |
| No. 2 | No.1 + 0.4 µg/mL hydrocortisone |
| No. 3 | No.2 + 400 µM non-essential amino acids |
| No. 4 | No.3 + 2 mM glutamine |
| No. 5 | No.4 + 7 µg/mL bovine pituitary extract |
| No. 6 | No.5 + 5 ng/mL insulin-like growth factor 1 |
| No. 7 | No.6 + 1:50 N2 additive |
| No. 8 | No.7 + 5% fetal bovine serum |

### (2) Obtaining and culture of primary cells of esophageal squamous carcinoma

### 1. Preparation process

The surfaces of 15 mL sterile centrifuge tube, pipette, 10 ml transfer pipette, sterile gun head, etc. were sterilized and placed into a ultra clean workbench for ultraviolet irradiation of 30 minutes. Take out the culture mediums prepared according to Table 1, the tissue digestive solution prepared according to Table 2 and the tissue cleaning solution prepared according to Table 3 were taken out from 4°C refrigerator 30 minutes in advance, and balanced to room temperature.

**Table 2 Fumulation of tissue digestive solution**

| **Components of tissue digestive solution** | **Addition volume (mL)** |
|---|---|
| HBSS (Gibco) | 250 |
| RPMI-1640 (Gibco) | 250 |
| collagenase II (Sigma) | 1 g |
| collagenase IV (Sigma) | 1 g |
| deoxyribonucleic acid I (Sigma) | 25000U |
| hyaluronidase (Sigma) | 250 mg |
| calcium chloride (Sigma) | 166.5 mg |
| bovine serum albumin (Sangon Biotech) | 5 g |
| Total (mL) | 500 |

**Table 3 Fumulation of tissue cleaning solution**

| **Components of tissue cleaning solution** | **Addition volume (mL)** |
|---|---|
| DMEM/F12 (Corning) | 488 |
| Primocin (Invitrogen) | 2 |
| penicillin / streptomycin solution double antibody (Gibco) | 10 |
| Total (mL) | 500 |

### 2. Isolation of primary cells of esophageal squamous carcinoma

2.1. The endoscopic tissue samples of esophageal cancer were obtained from the tissue samples of five informed and consenting esophageal cancer patients during endoscopic examination, namely Sample 50, Sample 51, Sample 52, Sample 53 and Sample 54, respectively. The endoscopic tissue was extracted in the ultra clean workbench and placed in a 15 mL centrifuge tube, which was then added with 5 mL tissue cleaning solution, and the resultant was mixed and rinsed once. The resultant was centrifuged at 1500 rpm for 4 minutes.
2.2. The supernatant was discarded, and the resultant was added with 1:1 mixture of basic culture medium and tissue digestive solution (the dosage is about 5 ml tissue digestive solution per 10 mg tissue). The sample was marked with name and number, sealed with sealing film, and then digested in a shakor at 37°C and 300 rpm. Whether the digestion is completed is determined via observation every 1 hour.
2.3. After digestion, undigested tissue blocks were filtered through a 40 µM filter screen. The tissue blocks on the filter screen were rinsed with the tissue cleaning solution. The residual cells were rinsed into a centrifuge tube and centrifuged at 1500 rpm for 4 minutes.
2.4. The supernatant was discarded and the remaining cell clusters were observed to determine whether they contain blood cells. If there are blood cells, 3 mL blood cell lysate (Sigma) was added, which was then mixed well, lysed at 4°C for 15 minutes, with shaking and mixing well once every 5 minutes. After lysis, the resultant was take out and centrifuged at 1500 rpm for 4 minutes.
2.5. The supernatant was discarded and the resultant was added with 2 mL basic culture medium to resuspend the cells for reserve.

### 3. Culture of primary cells of esophageal squamous carcinoma

3.1. Microscopic observation: 10 µL resuspended cells were drawn and plated on a glass slide, and the cancer cells were observed with a 10X objective lens under a microscope (EVOS M500, Invitrogen).
3.2. Live cell count: 10 µL of the resuspended cell suspension was fully mixed with 10 µL of trypan blue dye (Invitrogen), and then 10 µL of the mixture was added into a flow imaging counter (JIMBIO FIL, Jiangsu Jimbio Technology Co., Ltd.) to obtain the cell concentration, cell particle size and cell viability. Cells with particle size greater than 10 |jm were selected for counting.
3.3. Cell culture: The cell suspension from each sample, which has been counted in above 3.2, was devided into 9 equal parts respectively, and were centrifuged at 1500 rpm for 4 minutes, followed by resuspending with 200 µL BM and No. 1-8 medium respectively. The resultants were inoculated into 48-well plate at a living cell density of 1 × 10⁴/cm² (10,000 cells per well), and then the plate was added with NIH-3T3 cells that have been irradiated by γ-ray (irradiation dose 35Gy) at a cell density of 2 × 10⁴/cm². Finally, each well in the 48-well plate was supplemented to a volume of 500 µL with the corresponding culture mediums, and the resultant was fully mixed. After surface disinfection, the plate was placed in a 37°C, 5% CO₂ incubator (Thermo Fisher) for culture. After grew to cover more than 85% of the 48-well plate, the cells were passaged.

### (3) Results of culture

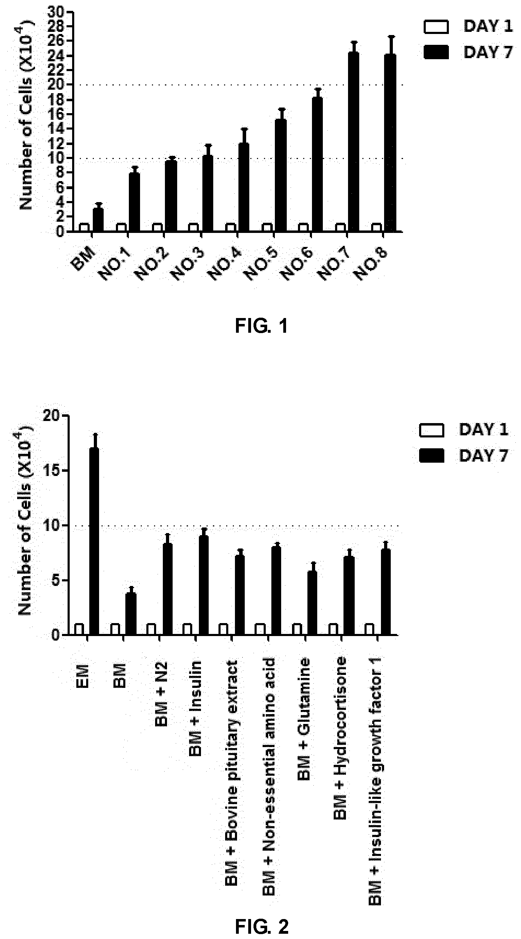

On the 7th day of culture, the 48-well plate was taken out, rinsed with 200 µL 0.25% trypsin (Gibco) for 1 minute. The liquid was removed by sucking, and 500 µL 0.05% trypsin (Gibco) was added to each well again. The resultant was placed in a 37°C, 5% CO₂ incubator to react for 10 minutes, and the digestion can be terminated until it can be observed under the microscope (EVOS M500, Invitrogen) that the cells have been completely digested. After being centrifuged at 1500 rpm for 4 minutes, the supernatant was discarded, and 1 mL of basic culture medium was added for resuspension. The total number of cells was obtained by counting with a flow imaging counter (JIMBIO FIL, Jiangsu Jimbio Technology Co., Ltd.). The results obtained from primary cells of esophageal squamous carcinoma isolated from endoscopic tissue Sample 50 are shown in Figure 1.

It can be known from the results shown in Figure 1, compared with the basic culture medium, the above No. 1-7 mediums can all promote the proliferation of primary cells of esophageal squamous carcinoma in different levels. When using the medium containing Y27632 and Primocin, insulin, hydrocortisone, non-essential amino acids, glutamine, bovine pituitary extract, IGF1, N2 additives (No. 7 medium) to culture primary cells of esophageal squamous carcinoma, the proliferation effect was significantly improved. In addition, when No. 8 medium obtained by adding 5% fetal bovine serum to No. 7 medium is used for the culture of primary cells of esophageal squamous carcinoma, the proliferation effect is not significantly improved compared with No. 7 medium without addition of 5% fetal bovine serum. Therefore, it can be concluded that the culture medium for primary cells of esophageal squamous carcinoma of the invention can be used without adding fetal bovine serum.

### Example 2. Effect of combination of multiple factors in the culture medium for primary cells of esophageal squamous carcinoma on the proliferation of primary cells of esophageal squamous carcinoma

To the basic culture mediums of Example 1 (DMEM/F12 medium + 10 µM Y27632 + 100 mg/mL Primocin) were separatedly added 1:50 ratio of N2 additive, 20 µg/mL insulin, 7 µg/mL bovine pituitary extract, 2 mM glutamine, 400 µM non-essential amino acids, 0.4 µg/mL hydrocortisone, 5 ng/mL insulin-like growth factor 1, respectively, to prepare the culture mediums of this Example.

According to the same process as in Example 1, endoscopic tissue Sample 57, Sample 58, Sample 59, Sample 60 and Sample 61 were isolated to obtain primary cells of esophageal squamous carcinoma, and the obtained primary cells of esophageal squamous carcinoma were cultured by using the respective culture mediums prepared in this Example, as well as BM medium and No. 7 medium (EM) of Example 1. The resultants were inoculated into 48-well plate at a living cell density of 1 × 10⁴/cm² (10,000 cells per well), and then the plate was added with NIH-3T3 cells that have been irradiated by γ-ray (irradiation dose 35Gy) at a cell density of 2 × 10⁴/cm², which were then fully mixed. After surface disinfection, the plate was placed in a 37°C, 5% CO₂ incubator (Thermo Fisher) for culture. On the 7th day of culture, the 48-well plate was taken out, rinsed with 200 µL 0.25% trypsin (Gibco) for 1 minute. The liquid was removed by sucking, and 500 µL 0.05% trypsin (Gibco) was added to each well again. The resultant was placed in a 37°C, 5% CO₂ incubator to react for 10 minutes, and the digestion can be terminated until it can be observed under the microscope (EVOS M500, Invitrogen) that the cells have been completely digested. After being centrifuged at 1500 rpm for 4 minutes, the supernatant was discarded, and 1 mL of basic culture medium was added for resuspension. The total number of cells was obtained by counting with a flow imaging counter (JIMBIO FIL, Jiangsu Jimbio Technology Co., Ltd.). The effect of combination of two factors in the culture medium for primary cells of esophageal squamous carcinoma on the proliferation of primary cells of esophageal squamous carcinoma was evaluated. The results obtained from primary cells of esophageal squamous carcinoma isolated from endoscopic tissue Sample 57 are shown in Figure 2.

As shown in Figure 2, when a factor selected from N2 additive, insulin, bovine pituitary extract, glutamine, non-essential amino acids, hydrocortisone, insulin-like growth factor 1 was further added into the basic culture medium (DMEM/F12 medium + 10 µM Y27632 + 100 mg/mL Primocin), the cell proliferation efficiency was better than that of the basic culture medium, but was not as good as that of the No. 7 medium (EM) in Example 1.

### Example 3. Effect of adding other factors to the culture medium for primary cells of esophageal squamous carcinoma of the invention on the proliferation of primary cells of esophageal squamous carcinoma

To No. 7 mediums (EM) of Example 1 were separately added 10 ng/ml fibroblast growth factor 2 (FGF2), 10 ng/ml fibroblast growth factor 10 (FGF10), 10 ng/ml fibroblast growth factor 7 (FGF7) or the combination of 10 ng/ml FGF2, 10 ng/ml FGF10 and 10 ng/ml FGF7, respectively, to prepare the mediums of this Example.

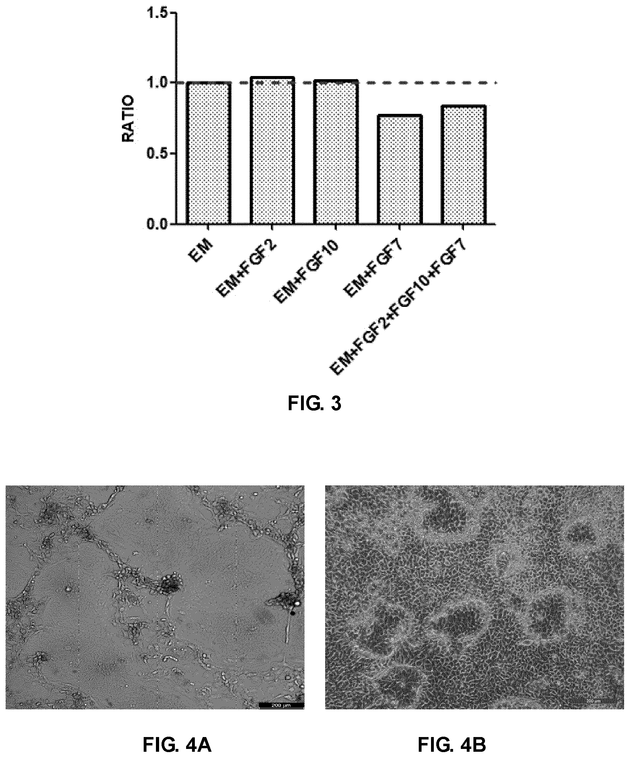

According to the same process as in Example 1, endoscopic tissue Sample 62 and Sample 63 were isolated to obtain primary cells of esophageal squamous carcinoma, and the obtained primary cells of esophageal squamous carcinoma were cultured by using the respective culture mediums prepared in this Example, as well as No. 7 medium (EM) of Example 1. The resultants were inoculated into 48-well plate with at a cell density of 1 × 10⁴/cm² (10,000 cells per well), and then the plate was added with NIH-3T3 cells that have been irradiated by γ-ray (irradiation dose 35Gy) at a cell density of 2 × 10⁴/cm², which were then fully mixed. After surface disinfection, the plate was placed in a 37°C, 5% CO₂ incubator (Thermo Fisher) for culture. On the 7th day of culture, the 48-well plate was taken out, rinsed with 200 µL 0.25% trypsin (Gibco) for 1 minute. The liquid was removed by sucking, and 500 µL 0.05% trypsin (Gibco) was added to each well again. The resultant was placed in a 37°C, 5% CO₂ incubator to react for 10 minutes, and the digestion can be terminated until it can be observed under the microscope (EVOS M500, Invitrogen) that the cells have been completely digested. After being centrifuged at 1500 rpm for 4 minutes, the supernatant was discarded, and 1 mL of No. 7 medium (EM) was added for resuspension. The total number of cells was obtained by counting with a flow imaging counter (JIMBIO FIL, Jiangsu Jimbio Technology Co., Ltd.). The effect of adding other factors to the culture medium for primary cells of esophageal squamous carcinoma of the invention on the proliferation of primary cells of esophageal squamous carcinoma was evaluated. The results obtained from primary cells of esophageal squamous carcinoma isolated from endoscopic tissue Sample 62 are shown in Figure 3.

In Figure 3, the ratio in the ordinate represents the ratio of the number of cells obtained by culturing for 7 days with the culture comprising EM medium and additional different factor(s) to the number of cells obtained by culturing for 7 days with EM medium. If the ratio is greater than 1, it indicates that the addition of different factor(s) to EM medium provides better effect for promoting proliferation. If the ratio is less than 1, it indicates that the addition of different factor(s) does not produce preferable effect than EM medium.

As shown in Fig. 3, when the mediums obtained by further adding FGF2 or FGF7 to No. 7 medium (EM) of Example 1 were used for culture, there is no significant effect for promoting proliferation as compared with EM medium. When the mediums obtained by further adding FGF7 to No.7 medium (EM) of Example 1 were used for culture, the proliferation of primary cells of esophageal squamous carcinoma was even inhibited as compared with EM medium.

### Example 4. Culture and identification of primary cells of esophageal squamous carcinoma

According to the same process as in Example 1, endoscopic tissue Sample 64 was isolated to obtain primary cells of esophageal squamous carcinoma, and the obtained primary cells of esophageal squamous carcinoma were cultured by using No. 7 medium (EM) of Example 1. The resultants were inoculated into 12-well plate at a living cell density of 1 × 10⁴/cm² (45,000 cells per well), and then the plate was added with NIH-3T3 cells that have been irradiated by γ-ray (irradiation dose 35Gy) at a cell density of 2 × 10⁴/cm², which were then fully mixed. After surface disinfection, the plate was placed in a 37°C, 5% CO₂ incubator (Thermo Fisher) for culture.

On Day 12th, the cultured esophageal squamous carcinoma cells were observed under the microscope (EVOS M500, Invitrogen). Figure 4A and Figure 4B are the photos taken under the 4X and 10X objective lens, respectively. As seen under the microscope, the cells are closely arranged and the morphologies are slightly irregular.

Next, the cultured cells were rinsed with 200 µL 0.25% trypsin (Gibco) for 1 minute. The liquid was removed by sucking, and 500 µL 0.05% trypsin (Gibco) was added to each well again. The resultant was placed in a 37°C, 5% CO₂ incubator to react for 10 minutes, and the digestion can be terminated until it can be observed under the microscope (EVOS M500, Invitrogen) that the cells have been completely digested. After being centrifuged at 1500 rpm for 4 minutes, the supernatant was discarded, and 500 µL of No. 7 medium (EM) was added for resuspension. The cultured esophageal squamous carcinoma cells were identified.

100 µL of the above esophageal squamous carcinoma cell suspension was coated on a glass slide and identified by Wright-Giemsa staining. The staining process is described below.
(1) The cell suspension smears were air dried, and then were dropped with 1 drop of Wright-Giemsa A solution (Baso), followed by 3 drops of Wright-Giemsa B solution (Baso), fully mixed and dyed for 3 minutes.
(2) The smears were rinsed with running water, noting that the dye solution should be removed by rinsing water instead of pouring out, so as to prevent sediment from depositing on the specimen.
(3) The smears were dried, then were observed and pictured under a microscope (Olympus CX41).

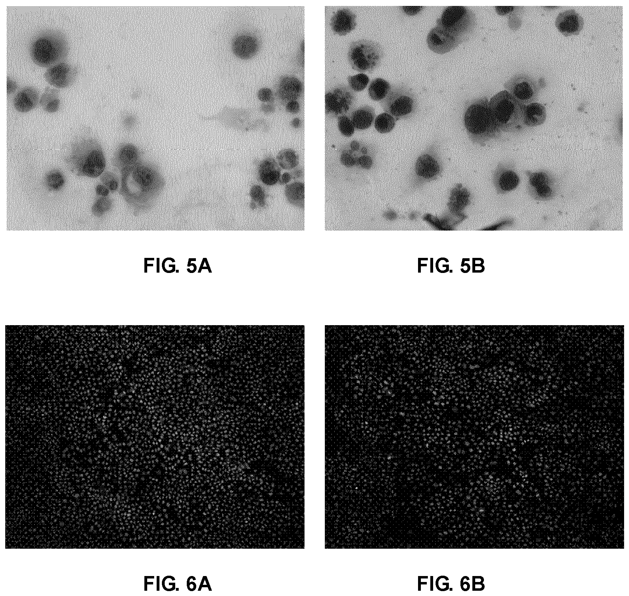

Figure 5A and Figure 5B show the identification results of esophageal squamous carcinoma cells cultured in vitro by Wright-Giemsa staining, which are the pictures taken in different fields under the 10X objective lens, respectively. The nucleus are large and deeply stained, which are consistent with the characteristics of squamous carcinoma cells.

The esophageal squamous carcinoma cells cultured from the sample were stained with immunofluorescence. The staining process is described below.

### (1) Adherent

The cultured esophageal squamous carcinoma cells were seeded on cell slides (Thermo Fisher) and cultured in a 37°C, 5% CO₂ incubator until the cells adhered to the wall.

### (2) Fixation

① PBS (Shanghai Sangon Biotech) was used to prepare 4% formaldehyde (Sigma), which was then stored in 4°C refrigerator for use.
② After the cells adhered to the wall, the culture solution was discarded and the cells were fixed on ice with 4% formaldehyde for 30 minutes. The resultant was washed with PBS (Shanghai Sangon Biotech) for 5 minutes X 3 times.

### (3) Transparentizing (avoiding light)

① Preparing transparentizing solution: PBS + 0.3% H₂O₂ (Shanghai Sangon Biotech) + 0.3% Triton X-100 (Shanghai Sangon Biotech).
② Transparentizing: PBS was discarded, and then the transparentizing solution was added. The resultant was shaked (about 100 rpm) without exposure to light to transparentize for 30 minutes, and was washed with PBS for 5 minutes X 3 times.

### (4) Blocking

PBS + 0.3% Triton X-100 to prepare 5% BSA (Shanghai Sangon Biotech) was used for blocking, and the blocking was conducted at 37°C for 30 minutes.

### (5) Primary antibody incubation

① PBS + 0.3% Triton X-100 was prepared to dilute the antibody, wherein the squamous carcinoma specific antibody p63 (CST) was diluted in a ratio of 1:50. discard the blocking solution was discarded, and the prepared primary antibody diluent was added. The resultant was incubated in a refrigerator at 4°C overnight.
② The resultant was taken out at 4°C, balanced to room temperature, continued for incubation at 37°C for 1 hour, and then washed with PBS for 5 minutes X 3 times.

### (6) Secondary antibody (avoiding light)

PBS + 0.3% Triton X-100 was prepared for secondary antibody dilution, wherein the rabbit fluorescent secondary antibody (Thermo Fisher) with excitation light of 488 was diluted in a ratio of 1:1000. The resultant was incubated at room temperature in the dark for 1 h, and then was washed with PBS for 5 minutes X 3 times.

### (7) DAPI staining (avoiding light)

1:1000 DAPI (Sigma) in PBS dilution was used for staining at room temperature in the dark for 5 minutes. The resultant was washed with PBS for 5 minutes X 3 times. Pictures were taken under microscope (EVOS M500, Invitrogen) for record.

Figure 6A and Figure 6B are the identification results of esophageal squamous carcinoma cells cultured in vitro by immunofluorescence staining, which are the fluorescent pictures taken in different fields under the 10X objective lens, respectively. As shown in the Figures, the cells in the visual field all display green under 488 excitation light, indicating that the cultured cells are squamous carcinoma cells, which are consistent with the clinical pathological diagnosis.

Example 6. Statistics of first culture period and cell number of esophageal squamous carcinoma and calculation of Population Doubling (PD) value

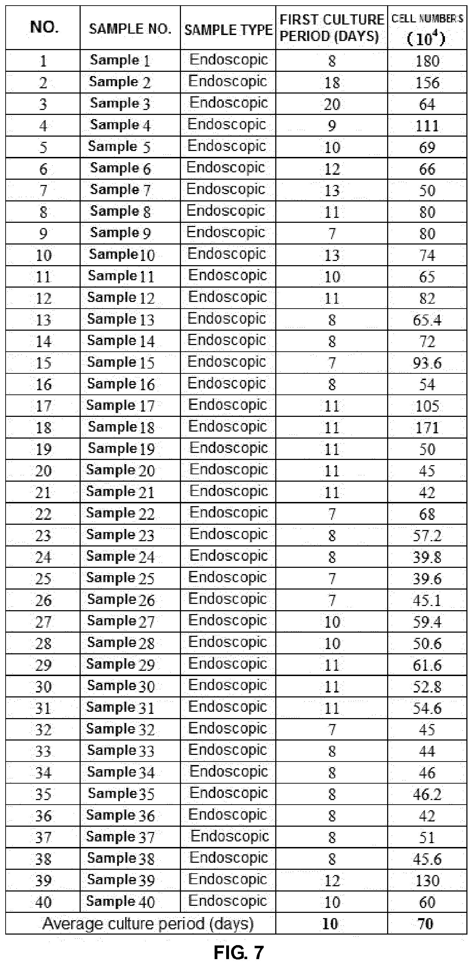

According to the same process as in Example 1, endoscopic tissue samples of esophageal carcinoma (Samples 1-40) were digested to obtain the primary cells of esophageal squamous carcinoma. The obtained primary cells of esophageal squamous carcinoma were cultured by using No. 7 medium in Example 1. The cells were inoculated into 12-well plate at a living cell density of 1 × 10⁴/cm² for culture. After expanded to cover 85% of the plate, the cells were digested and counted. At the same time, the days of culture until digestion, which were taken as one culture period, were recorded. As shown in Figure 7, the average culture period of 40 samples was 10 days, and the average number of cells expanded was 700,000.

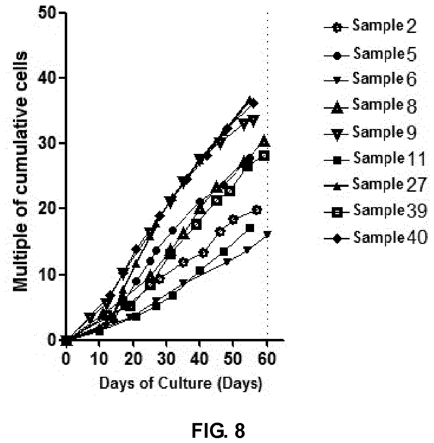

Under this experimental condition, a total of 9 samples, i.e. Sample 2, Sample 5, Sample 6, Sample 8, Sample 9, Sample 11, Sample 27, Sample 39 and Sample 40, were continuously cultured. The expanded cells were amplified in different passages. Each passage was counted after digestion and the corresponding culture period was recorded. PD value was calculated according to the formula, Population Doubling (PD) = 3.32*log10 (total number of cells after digestion / initial number of inoculated cells). As shown in Figure 8, in which the abscissa represents the days of cell culture, and the ordinate represents the multiple of cumulative cell proliferation, i.e., the multiple of cell expansion in the culture period. The larger the value, the more multiples the cell expands in certain period, that is, the more cells are expanded. The slope represents the rate of cell expansion.

It can be seen from Figure 8 that when the above 9 samples were cultured by using the culture medium for primary cells of esophageal squamous carcinoma of the invention, the cell expansion rate remains substantially unchanged after 56 days of amplification, and the ability of continuous expansion was still achieved.

### Example 7. Effects of concentrations of the added factors on the proliferation of esophageal squamous carcinoma cells

According to the same process as in Example 1, endoscopic tissue Sample 65, Sample 66, Sample 67, Sample 68 and Sample 69 were isolated to obtain primary cells of esophageal squamous carcinoma, and the obtained primary cells of esophageal squamous carcinoma were cultured by using No. 7 medium of Example 1. The resultants were inoculated into 12-well plate at a living cell density of 1 × 10⁴/cm² (45,000 cells per well), and then the plate was added with NIH-3T3 cells that have been irradiated by γ-ray (irradiation dose 35Gy) at a cell density of 2 × 10⁴/cm², which were then fully mixed. After surface disinfection, the plate was placed in a 37°C, 5% CO₂ incubator (Thermo Fisher) for culture. When the cells expended to cover 85% of the plate, the 12-well plate was taken out, rinsed with 200 µL 0.25% trypsin (Gibco) for 1 minute. The liquid was removed by sucking, and 500 µL 0.05% trypsin (Gibco) was added to each well again. The resultant was placed in a 37°C, 5% CO₂ incubator to react for 10 minutes, and the digestion can be terminated until it can be observed under the microscope (EVOS M500, Invitrogen) that the cells have been completely digested. After being centrifuged at 1500 rpm for 4 minutes, the supernatant was discarded, and 1 mL of basic culture medium was added for resuspension. The total number of cells was obtained by counting with a flow imaging counter (JIMBIO FIL, Jiangsu Jimbio Technology Co., Ltd.). The obtained cells were used for the following cultivation experiments.

Next, the following 8 medium with different formulations were prepared for experiments:
Formulation 1: No. 7 medium of Example 1 without N2 additive;
Formulation 2: No. 7 medium of Example 1 without non-essential amino acids;
Formulation 3: No. 7 medium of Example 1 without glutamine;
Formulation 4: No. 7 medium of Example 1 without insulin;
Formulation 5: No. 7 medium of Example 1 without IGF1;
Formulation 6: No. 7 medium of Example 1 without bovine pituitary extract;
Formulation 7: No. 7 medium of Example 1 without Y27632;
Formulation 8: No. 7 medium of Example 1 without hydrocortisone.

The digested cell suspension was diluted with the above Formulations 1-8 and No.7 medium in Example 1 respectively, and were seeded into a 24-well plate with 20,000 cells and 500 µL volume per well.

When using the medium of Formulation 1, five concentration gradients of N2 additives with the final concentration of 1:400, 1:200, 1:100, 1:50 and 1:25 were prepared, respectively, and the prepared N2 additives were added respectively to a 24-well plate inoculated with primary cells, with 500 µL per well; the medium of Formulation 1 was used as the control hole (BC).

When using the medium of Formulation 2, five concentration gradients of non-essential amino acids with the final concentration of 400 µM, 200 µM, 100 µM, 50 µM, 25 µM were prepared, respectively, and the prepared non-essential amino acids were added respectively to a 24-well plate inoculated with primary cells, with 500 µL per well; the medium of Formulation 2 was used as the control hole (BC).

When using the medium of Formulation 3, five concentration gradients of glutamine with the final concentration of 8 mM, 4 mM, 2 mM, 1 mM, 0.5 mM were prepared, respectively, and the prepared glutamine were added respectively to a 24-well plate inoculated with primary cells, with 500 µL per well; the medium of Formulation 3 was used as the control hole (BC).

When using the medium of Formulation 4, five concentration gradients of insulin with the final concentration of 40 µg/mL,20 µg/mL,10 µg/mL, 5 µg/mL,2.5 µg/mL were prepared, respectively, and the prepared insulin were added respectively to a 24-well plate inoculated with primary cells, with 500 µL per well; the medium of Formulation 4 was used as the control hole (BC).

When using the medium of Formulation 5, five concentration gradients of insulin-like growth factor 1 (IGF1) with the final concentration of 40 ng/mL, 20 ng/mL, 10 ng/mL, 5 ng/mL, 2.5 ng/mL were prepared, respectively, and the prepared insulin-like growth factor 1 (IGF1) were added respectively to a 24-well plate inoculated with primary cells, with 500 µL per well; the medium of Formulation 5 was used as the control hole (BC).

When using the medium of Formulation 6, five concentration gradients of bovine pituitary extract with the final concentration of 56 µg/mL, 28 µg/mL, 14 µg/mL, 7 µg/mL, 3.5 µg/mL were prepared, respectively, and the prepared bovine pituitary extract were added respectively to a 24-well plate inoculated with primary cells, with 500 µL per well; the medium of Formulation 6 was used as the control hole (BC).

When using the medium of Formulation 7, five concentration gradients of Y27632 with the final concentration of 40 µM, 20 µM, 10 µM, 5 µM, 2.5 µM were prepared, respectively, and the prepared Y27632 were added respectively to a 24-well plate inoculated with primary cells, with 500 µL per well; the medium of Formulation 7 was used as the control hole (BC).

When using the medium of Formulation 8, five concentration gradients of hydrocortisone with the final concentration of 1.6 µg/mL,0.8 µg/mL,0.4 µg/mL,0.2 µg/mL, 0.1 µg/mL were prepared, respectively, and the prepared hydrocortisone were added respectively to a 24-well plate inoculated with primary cells, with 500 µL per well; the medium of Formulation 8 was used as the control hole (BC).

In addition, 8 small molecule compounds (LDN193189, IWP2, A83-01, Forskolin, SB431542, CHIR99021, DMH-1, DAPT; all purchased from MCE) with 5 concentration gradients were prepared in No. 7 medium of Examples to give final concentration of 10 µM, 3 µM, 1 µM, 0.3 µM, 0.1 µM, respectively, and the prepared 8 small molecule compounds (LDN193189, IWP2, A83-01, Forskolin, SB431542, CHIR99021, DMH-1, DAPT) were added respectively to 24-well plates inoculated with primary cells, with 500 µL per well; the No. 7 medium was used respectively as the control hole (BC).

At the same time, 10,000 irradiated NIH-3T3 cells were added to each of the above holes as trophoblastic cells.

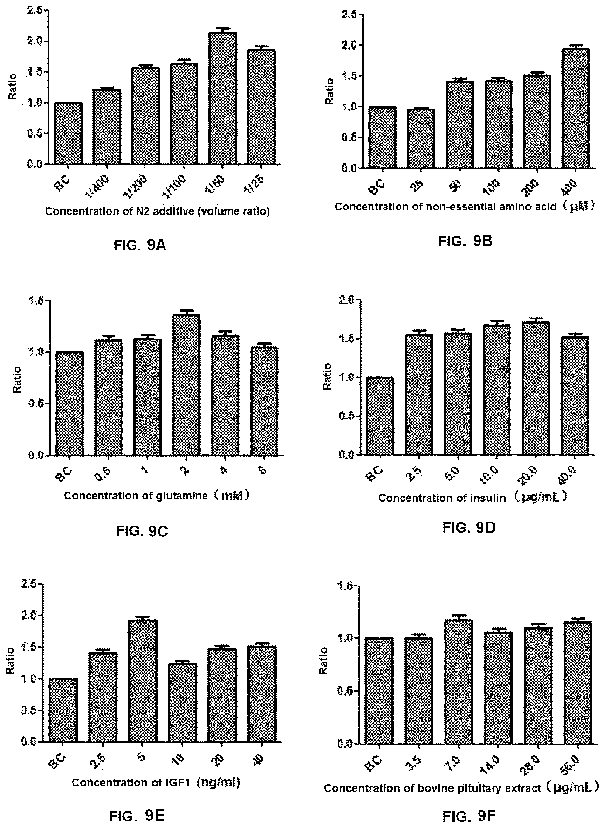

After the cells were expanded to about 85% of the 24 wells, the cells were digested and counted, the ratios were calculated by referring to the cell numbers in the control well (BC), and the results were shown in Figures 9A- 9P. In each of Figures 9A-9P, the ratio represents a ratio of the number of cells of the first passage cultured by using each culture medium to the number of cells of the first passage cultured by the corresponding control hole. If the ratio is greater than 1, it indicates that the proliferation promoting effect of the prepared medium containing different concentrations of factors or small molecular compounds is preferable over that of the control well medium; if the ratio is less than 1, it indicates that the proliferation promoting effect of the prepared medium containing different concentrations of factors or small molecular compounds is poorer than that of the control well medium.

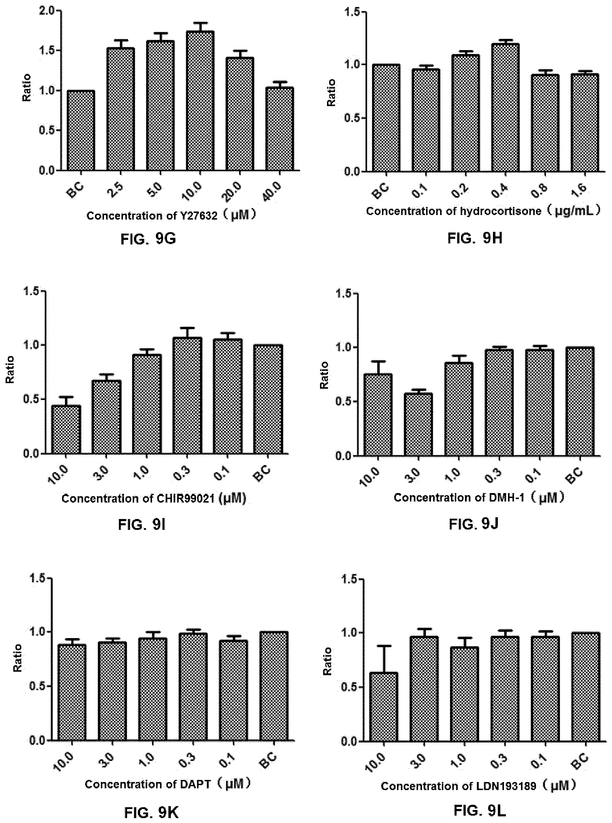

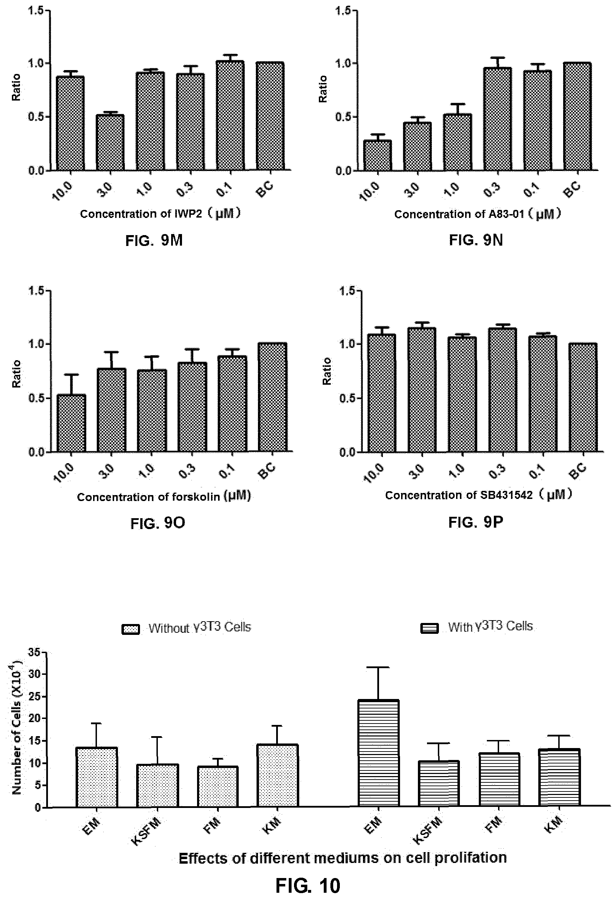

As shown in Figure 9, the concentration range of N2 additive is 1:400-1:25, and the cell proliferation effect is most significant when adding in a ratio of 1:50; the concentration range of non-essential amino acids is 25-400 µM, and the cell proliferation effect is most significant when adding in a concentration of 400 µM; the concentration range of glutamine is 0.5-8 mM, and the cell proliferation effect is most significant when adding in a concentration of 2 mM; the concentration range of insulin-like growth factor 1 is 2.5-40 ng/mL, and the cell proliferation effect is most significant when the concentration is 5 ng/mL; the concentration range of bovine pituitary extract is 3.5-56 µg/mL, and the cell proliferation effect is most significant when the concentration is 7 µg/mL; the concentration range of Y27632 is 2.5-40 µM, and the cell proliferation effect is most significant when the concentration is 10 µM; the concentration range of insulin is 2.5-40 ng/mL, and the cell proliferation effect is most significant when the concentration is 20 ng/mL; the concentration range of hydrocortisone is 0.1-1.6 µg/mL, and the cell proliferation effect is most significant when the concentration is 0.4 µg/mL. Other 8 small molecule compounds (LDN193189, IWP2, A83-01, Forskolin, SB431542, CHIR99021, DMH-1, DAPT) do not show significant proliferation promoting effect at the concentration gradients of 10 µM, 3 µM, 1 µM, 0.3 µM, 0.1 µM.

### Example 8. Comparison of the effect of the culture medium of the invention and the prior art medium on cell proliferation

KM (Keratinocyte medium) medium of non-patent document 2 was prepared as Table 4.

**Table 4. Preparation of KM medium**

| **Components of Medium** | **Addition Volume (mL)** |
|---|---|
| DMEM (Corning) | 329.25 |
| F12 (Corning) | 109.75 |
| Insulin (Gibco) | 5 |
| Epidermal growth factor (Sino Biological) | 0.5 |
| Linothyronine (mce) | 0.005 |
| Transferrin (Sino Biological) | 0.5 |
| Cholera toxin (sigma) | 0.005 |
| Hydrocortisone (mce) | 5 |
| Fetal bovine serum (ExCell Biotech Co., Ltd.) | 50 |
| Total (mL) | 500 |

FM medium of non-patent document 3 was prepared as Table 5.

**Table 5. Preparation of FM medium**

| **Components of Medium** | **Addition Volume (mL)** |
|---|---|
| DMEM (Corning) | 373 |
| F12 (Corning) | 125 |
| Y27632 (mce) | 0.5 |
| Insulin (Gibco) | 1.25 |
| Epidermal growth factor (Sino Biological) | 0.02 |
| Cholera toxin (sigma) | 0.0043 |
| Amphotericin B (sigma) | 0.5 |
| Hydrocortisone (mce) | 0.03 |
| Total (mL) | 500 |

KSFM medium was purchased from StemCell.

According to the same process as in Example 1, endoscopic tissue Sample 70, Sample 71 and Sample 72 were isolated to obtain primary cells of esophageal squamous carcinoma, which were cultured by using the EM medium, KSFM medium, FM medium and KM medium, respectively.

One of the samples (Sample 70) is described below. The obtained primary cells of esophageal squamous carcinoma were resuspended with 1 mL EM medium, KSFM medium, FM medium and KM medium, respectively, and were counted with a flow imaging counter (JIMBIO FIL, Jiangsu Jimbio Technology Co., Ltd.).

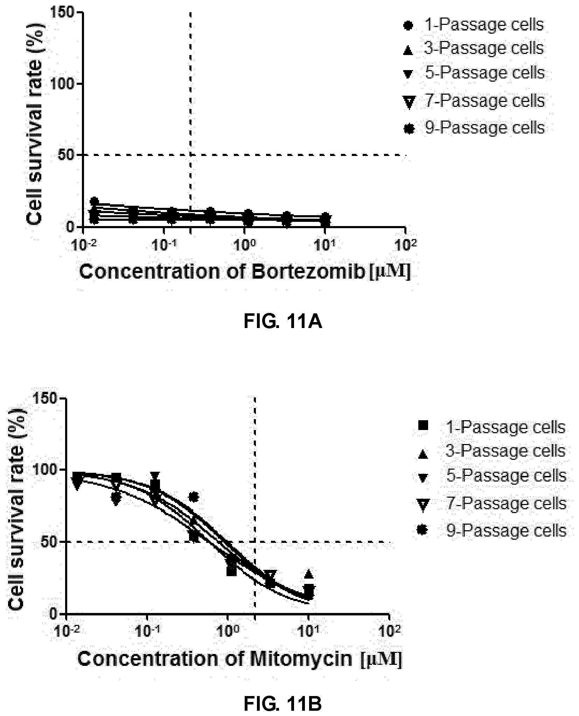

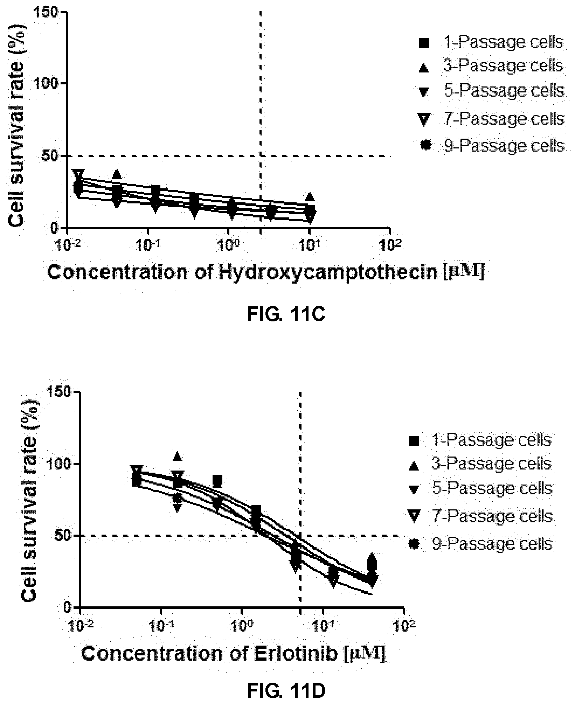

The resultants were inoculated into two 24-well plates at a living cell density of 1 × 10⁴/cm² (20,000 cells per well), respectively, wherein one 24-well plate was cultured without addition of NIH-3T3 cells that have been irradiated by γ-ray and the other 24-well plate was added with NIH-3T3 cells that have been irradiated by γ-ray (irradiation dose 35Gy) at a cell density of 2 × 10⁴/cm², which were then fully mixed. After surface disinfection, the plates were placed in a 37°C, 5% CO₂ incubator (Thermo Fisher) for culture. On the 5th day of culture, the 24-well plates were taken out, rinsed with 200 µL 0.25% trypsin (Gibco) for 1 minute. The liquid was removed by sucking, and 500 µL 0.05% trypsin (Gibco) was added to each well again. The resultants were placed in a 37°C, 5% CO₂ incubator to react for 10 minutes, and the digestion can be terminated until it can be observed under the microscope (EVOS M500, Invitrogen) that the cells have been completely digested. After being centrifuged at 1500 rpm for 4 minutes, the supernatant was discarded, and 1 mL of basic culture medium was added for resuspension. The total numbers of cells cultured by different mediums were obtained by counting with a flow imaging counter (JIMBIO FIL, Jiangsu Jimbio Technology Co., Ltd.). The results are shown in Figure 11.

It can be seen from Figure 10 that, compared with KM medium, FM medium and KSFM medium, EM medium can obtain the largest number of expanded cells under the condition of inoculation with the same number of cells (20,000/well) and culture for the same time, and thus, has a significant effect on promoting the proliferation of esophageal squamous carcinoma cells. In addition, it can also be seen from Figure 10 that the effect of EM medium in culture with the use of trophoblast cells is better than that in the absence of trophoblast cells. Thus, the culture medium for primary cells of esophageal squamous carcinoma of the invention has a significant effect on promoting the proliferation of esophageal squamous carcinoma cells when culturing with trophoblast cells, which may shorten the period for culture.

### Example 9. Use of the primary cells of esophageal squamous carcinoma obtained by expanding from the culture medium of the invention in drug screening

### 1. Culture and plating of cells

According to the same process as in Example 1, primary cells of esophageal squamous carcinoma were isolated from the endoscopic samples of esophageal squamous carcinoma, and cultured with No. 7 medium of Example 1. After the cells were expanded to cover 85% of the plate, they were digested as one passage. The first, third, fifth, seventh and ninth passages of the cultured cells were selected for drug screening.

The cells were digested and counted according to the steps in Example 1, and were fully mixed with No. 7 medium of Example 1 in a loading slot (Corning) at a living cell density of 4 × 10⁴ cells/cm², and then cultured in a 384-well opaque white cell culture plate (Corning). The volume of each well was 50 µL and the number of cells was 2,000/well. The plate was sealed by adding No. 7 medium of Example 1 from the edge of the plate, and the sample names and testing times of CellTiter-Glo (Promega) were marked on the plate. The surface was disinfected with 75% alcohol (LIRCON), and the resultant was cultured in a 37°C, 5% CO₂ incubator. The drugs were added after 24 hours.

### 2. Preparation of candidate drugs

Three drugs (Bortezomib, Mitomycin and Hydroxycamptothecin; all purchased from MCE) in 7 concentration gradients were prepared according to the following table, which were each added to each well of a 384-well plate (Thermo Fisher) in a volume of 50 µL and stored at -20°C for use.

**Table 6. Preparation of drug solutions of Bortezomib, Mitomycin, Hydroxycamptothecin**

| Final concentration (µM) | Concentration of prepartion solution (µM) |
|---|---|
| 10 | 5000 |
| 3.33 | 1666.7 |
| 1.11 | 555.6 |
| 0.37 | 185.2 |
| 0.12 | 61.7 |
| 0.04 | 20.6 |
| 0.01 | 6.9 |

Erlotinib (MCE) in 7 concentration gradients were prepared according to the following table, which were each added to each well of a 384-well plate (Thermo Fisher) in a volume of 50 µL and stored at -20°C for use.

**Table 7. Preparation of Erlotinib solution**

| Final concentration (µM) | Concentration of prepartion solution (µM) |
|---|---|
| 40 | 20000 |
| 13.33 | 6666.7 |
| 4.44 | 2222.2 |
| 1.48 | 740.7 |
| 0.49 | 246.9 |
| 0.16 | 82.3 |
| 0.05 | 27.4 |

### 3. High-throughput drug addition

The prepared drug plate was taken out and standed at room temperature. After completely melted, the plate was placed in a centrifuge (Sigma 3-18K) and centrifuged at room temperature for 1 minute at 1,000 rpm. A high-throughput automated workstation (JANUS, Perkin Elmer) was used for high-throughput dosing. To each well of the 384-well plate with cultured esophageal squamous carcinoma cells was added 0.1 µL of the candidate drugs of corresponding concentrations. After dosing, the surface of 384-well plate was disinfected and moved to the incubator for further culture. The cell viability was measured after 72 hours.

### 4. Measurment of cell viability

CellTiter-Glo luminescent reagent (Promega) was taken out from a 4 °C refrigerator, and 10 mL of the reagent was added into the loading slot; the 384-well plate for testing was taken out from the incubator, and 10 µL CellTiter-Glo luminescent reagent was added into each well. After being standed for 10 mins, the test was conducted by using a multi-functional microplate reader (Envision, Perkin Elmer).

### 5. Data processing

According to the formula, Cell survival rate (%) = Chemiluminescence value of drug well / Chemiluminescence value of control well *100%, the cell survival rate of cells treated with different drugs was calculated, and the half-inhibition rate (IC₅₀) of drugs on cells was calculated by using graphpad prism software. The results are shown in Figures 11A-11D, respectively.

It can be confirmed from Figures 11A-11D that when the esophageal squamous carcinoma cells cultured from the culture medium for primary cells of esophageal squamous carcinoma of the invention were used for drug screening, the inhibitory effects of the same drug on the cultured cells of different passages remain substantially the same (the inhibition curve are substantially consistent).

### Industrial applicability

The invention provides a culture medium and a cultivation method for rapidly expanding primary cells of esophageal squamous carcinoma in vitro, which can realize the rapid expansion of tissue samples of esophageal squamous carcinoma in a short time, and amplify sufficient amounts of cells in an effective time, which can be used for in vitro high-throughput drug sensitivity tests and for the guidance of precise clinical medication.

## Claims

1. A culture medium for primary cells of esophageal squamous carcinoma, **characterized in** comprising:
an initial culture medium, Rho protease inhibitor, antibiotic, insulin, N2 additive, insulin-like growth factor 1, non-essential amino acid, and optionally hydrocortisone, optionally glutamine, and optionally bovine pituitary extract,
the initial culture medium is selected from DMEM/F12, DMEM, F12 or RPMI-1640.

2. The culture medium for primary cells of esophageal squamous carcinoma of claim 1, **characterized in that**:
the Rho protease inhibitor is selected from one or more of Y27632, Hydroxyfasudil and GSK429286A; in case of Y27632, having a concentration within the range of 2.5-40 µM, preferably 5-20 µM; in case of Hydroxyfasudil, having a concentration within the range of 2-32 µM, preferably 4-16 µM; and in case of GSK429286A, having a concentration within the range of 2-32 µM, preferably 4-16 µM;
the antibiotics is selected from one or more of streptomycin / penicillin, Amphotericin B and Primocin; in case of streptomycin / penicillin, with streptomycin having a concentration within the range of 25-400 µg/mL,preferably 50-200 µg/mL, with penicillin having a concentration within the range of 25-400 U/mL, preferably 50-200 U/mL, more preferably 200 U/mL; in case of Amphotericin B, having a concentration within the range of 0.25-4 µg/mL,preferably 0.5-2 µg/mL; and in case of Primocin, having a concentration within the range of 25-400 mg/mL, preferably 50-200 mg/mL;
the insulin has a concentration within the range of 2.5-40 µg/mL, preferably 10-40 µg/mL;
the N2 additive has a volume ratio to the culture medium of 1:400-1:25, preferably 1:100-1:25;
the insulin-like growth factor 1 has a concentration within the range of 2.5-40 ng/mL, preferably 2.5-10 ng/mL;
the non-essential amino acid is selected from one or more of glycine, alanine, asparagine, aspartic acid, glutamic acid, proline and serine, with a total concentration within the range of 50-400 µM, preferably 100-400 µM;
the hydrocortisone has a concentration within the range of 0-1.6 µg/mL, preferably 0.2-0.8 µg/mL;
the glutamine has a concentration within the range of 0-8 mM, preferably 1-4 mM;
the bovine pituitary extract has a concentration within the range of 0-56 µg/mL, preferably 3.5-14 µg/mL.

3. A cultivation method of primary cells of esophageal squamous carcinoma, **characterized in that**:
primary cells of esophageal squamous carcinoma are cultured by using the culture medium for primary cells of esophageal squamous carcinoma according to claim 1 or 2.

4. The cultivation method of claim 3, **characterized in that**:
in the culture process, trophoblastic cells are added at a cell density of 2-3 × 10⁴/cm².

5. The cultivation method of claim 4, **characterized in that**:
the trophoblastic cells are irradiated NIH-3T3 cells, and the irradiation source is X-ray or γ-ray, with radiation dose of 30-50 Gy.

6. A method for screening drugs for esophageal squamous carcinoma, **characterized in** comprising the following steps:
(1) culturing primary cells of esophageal squamous carcinoma for drug screening, by using the cultivation method of primary cells of esophageal squamous carcinoma according to any one of claims 3-5;
(2) Selecting the drug to be tested and diluting the drug based on required concentration gradients;
(3) adding the diluted drug to the cells cultured and obtained in step (1);
(4) measuring the cell viability.
